**Europäisches Patentamt**

(19) **European Patent Office**

**Office européen des brevets**

(11) Numéro de publication: **0 079 861**
**B1**

(12) FASCICULE DE BREVET EUROPEEN

(45) Date de publication du fascicule du brevet:
04.09.85

(21) Numéro de dépôt: **82810488.5**

(22) Date de dépôt: **12.11.82**

(51) Int. Cl.⁴: **G 01 N 33/48,** G 01 N 35/00,
G 01 N 21/25

(54) Appareil pour déterminer le groupe sanguin d'un individu.

(30) Priorité: **18.11.81 CH 7400/81**

(43) Date de publication de la demande:
**25.05.83 Bulletin 83/21**

(45) Mention de la délivrance du brevet:
**04.09.85 Bulletin 85/36**

(84) Etats contractants désignés:
**AT BE CH DE FR GB IT LI LU NL SE**

(56) Documents cités:
**EP - A - 0 056 563**
**FR - A - 1 539 674**
**FR - A - 2 192 708**
**FR - A - 2 314 491**
**FR - A - 2 350 393**
**US - A - 3 645 690**
**US - A - 4 079 393**

(73) Titulaire: **Société FINAMEX Finance Corporation, Calle Aquilino de la Guardia, 8 Apartado 850, Ciudad de Panama (PA)**

(72) Inventeur: **Hubeau, Jean-Pierre, Rue de la Montagne 143, B-6110 Montigny-le-Tilleul (BE)**

(74) Mandataire: **Nithardt, Roland, CABINET ROLAND NITHARDT Rue Edouard Verdan 15, CH-1400 Yverdon (CH)**

ACTORUM AG

## Description

La présente invention concerne un appareil pour déterminer le groupe sanguin d'un individu, comportant un support d'analyse individuel transparent pourvu d'une rangée transversale de cavités scellées renfermant des réactifs liquides d'agglutination et monté sur une plaque porte-support, un dispositif équipé de pompes et d'un groupe mobile d'aiguilles de distribution pour prélever des échantillons de sang et les mélanger en quantités déterminées avec lesdits réactifs, des organes mécaniques pour assurer le déplacement des aiguilles de distribution, des organes d'analyse photométrique pour détecter la présence ou l'absence d'une réaction d'agglutination entre les échantillons sanguins et les réactifs, et un dispositif pour afficher les résultats de cette analyse.

On connaît déjà des appareils de ce type, notamment un appareil décrit dans la publication EP-A 056563, dans lequel un bras mobile porte des pipettes pour prélever des échantillons de sang et les déposer dans des cavités d'un support d'analyse individuel. Dans cet appareil, le mélange des réactifs avec les échantillons de sang se fait par écrasement d'une poche contenant initialement les réactifs, entre deux rouleaux tournant dans le même sens. Ce processus ne présente pas les caractères de fiabilité requis pour ce type d'analyse.

En ce qui concerne la détection de la réaction d'agglutination, ledit appareil utilise un procédé de mesure de la densité optique du mélange sang — antisérum qui manque de précision, en particulier parce que l'agglutination est plus ou moins intense selon les échantillons analysés, de sorte qu'il n'est que difficilement possible, voire impossible, de déterminer avec précision un seuil quantitatif de la densité optique du mélange.

Par ailleurs, dans tous les appareils connus, le problème du mélange homogène du sang et des réactifs n'est guère résolu d'une manière satisfaisante, de sorte que les analyses aboutissent parfois, dans des cas critiques, à des résultats erronés.

La présente invention se propose de pallier l'ensemble des inconvénients susmentionnés et d'apporter divers perfectionnements aux appareils connus, les rendant plus précis et plus fiables.

Dans ce but, l'appareil susmentionné est caractérisé en ce que le support d'analyse individuel comporte une rangée transversale de cavités d'analyse, dont chacune est alignée longitudinalement avec l'une desdites cavités pour réactifs, et au moins une cavité pour le sang, en ce que la plaque porte-support est mobile horizontalement dans la direction longitudinale du support d'analyse et le groupe d'aiguilles de distribution est mobile uniquement verticalement pour prélever le sang et les réactifs dans les cavités respectives et les déposer dans les cavités d'analyse, et en ce que les organes d'analyse photométrique comprennent un dispositif d'analyse colorimétrique comportant une série de détecteurs associés à des faisceaux lumineux respectifs qui traversent lesdites cavités d'analyse pendant que le support d'analyse est en déplacement.

Par rapport aux dispositifs connus, l'appareil selon l'invention présente une grande fiabilité, car il garantit le mélange d'une dose précise de sang, prélevée dans la cavité pour sang, avec une dose précise de chacun des réactifs dans un support individuel qui renferme par avance les réactifs aux emplacements voulus. En outre, grâce au déplacement des cavités d'analyse dans le dispositif colorimétrique, la détection d'une agglutination est facilitée. Enfin, les mouvements des organes mobiles sont uniquement des translations rectilignes, réalisables par des mécanismes simples et fiables.

La présente invention sera mieux comprise en référence à la description d'un exemple de réalisation préféré et au dessin annexé, dans lequel:

les fig. 1A et 1B représentent respectivement une vue d'ensemble extérieure de l'appareil selon l'invention et un schéma d'implantation des différents composants;

la fig. 2 représente une première forme de réalisation préférée du dispositif d'analyse tel qu'il est utilisé dans l'appareil de la fig. 1;

la fig. 3 représente une autre forme de réalisation du support d'analyse de la fig. 2;

la fig. 4 représente une variante du support d'analyse des fig. 2 et 3;

la fig. 5 est une vue en plan de la plaque portant les supports d'analyse des fig. 2 à 4;

les fig. 6A et 6B sont deux vues en élévation du mécanisme porte-aiguilles;

la fig. 7 est une vue en élévation du bloc de pompes;

la fig. 8 est une vue schématique du dispositif colorimétrique, et

la fig. 9 représente une vue d'un épiscope de renvoi de l'image des résultats d'analyse sur l'écran.

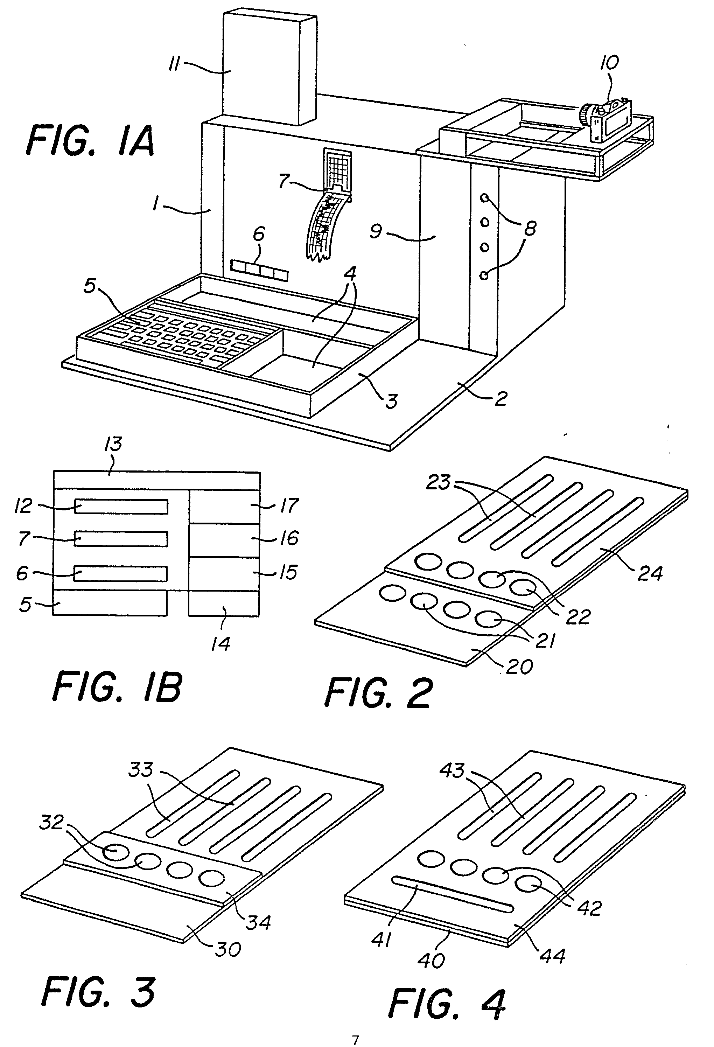

En référence aux fig. 1A et 1B, l'appareil pour déterminer le groupe sanguin d'un individu tel que décrit comporte un boîtier 1 pourvu d'un couvercle 2 rabattable, qui est amené en position lorsque l'appareil est prêt à l'emploi. Sur le couvercle est adapté un cadre 3 délimitant plusieurs cavités de rangement 4 et un clavier 5 de commande des fonctions. Le boîtier 1 contient l'ensemble des composants décrits en référence à la fig. 1B. Sur la face avant du boîtier, on aperçoit le dispositif d'affichage 6 des fonctions commandées par le clavier 5, l'imprimante 7, un ensemble 8 de voyants lumineux correspondant aux différentes fonctions et une ouverture 9 permettant l'accès aux supports d'analyse. Sur le dessus de l'appareil est monté un appareil photographique 10, un écran d'affichage des résultats d'analyse étant logé dans un boîtier 11, à côté duquel est prévu un support d'appui du menton du patient au moment de la prise de la photographie. Les composants représentés par la fig. 1B sont: le clavier de commande 5, le dispositif d'affichage 6, l'imprimante 7, l'enregistreur graphique 12, le bloc photographique 13 se composant des éléments 10 et 11, l'ensemble 14 portant les supports d'analyse, le bloc d'aiguilles 15, le dispositif d'analyse colorimétrique et l'épiscope 16 et le bloc de pompes 17. Ces différents composants sont décrits plus en détail par la suite.

La fig. 2 représente une forme de réalisation avantageuse du support d'analyse tel qu'il est utilisé dans l'appareil ci-dessus pour la détermination du groupe sanguin et du facteur rhésus d'un patient. Le support d'analyse représenté comporte une feuille 20 en un matériau thermoformable, sur laquelle sont ménagées une première série de cavités 21 et une seconde série de cavités 22, disposées en rangées transversales par rapport à la dimension longitudinale de la feuille 20. Les cavités de la première rangée sont destinées à recevoir chacune une quantité déterminée du sang d'un patient. Les cavités de la seconde rangée sont destinées chacune à recevoir un antisérum utilisé pour la détermination du groupe sanguin A, B, AB ou 0, ainsi que pour la détermination du facteur rhésus + ou −. Une ou plusieurs cavités supplémentaires par rangée peuvent être prévues pour la détermination de caractéristiques supplémentaires du facteur rhésus.

Des cavités d'analyse 23, sous forme de canaux, s'étendent longitudinalement le long d'une partie de la feuille 20 et sont disposées parallèlement entre elles et dans le prolongement de la ligne médiane reliant une cavité 21 et la cavité 22 correspondante. Ces cavités d'analyse servent de récipient dans lesquels on mélange le sang du patient avec les antisérums utilisés, et dans lesquels est censée s'effectuer la réaction d'agglutination.

Les cavités 21, 22 et 23 sont réalisées par déformation de la feuille 20, dont le matériau thermoplastique est choisi de façon à demeurer inerte vis-à-vis du sang et des antisérums utilisés. La feuille 20 est de préférence transparente, pour que les coagulations éventuelles, provoquées par la réaction du sang et des antisérums dans les cavités d'analyse 23, puissent être observées soit directement soit à travers la feuille 20.

Une pellicule d'occlusion 24 est de préférence thermosoudée sur une partie de la feuille 20, de manière à obturer de façon étanche au moins les cavités 22 contenant les antisérums, mais pouvant également obturer les cavités d'analyse 23. Les supports d'analyse des fig. 2 à 3 sont prêts à l'emploi, c'est-à-dire munis des antisérums utilisés pour l'analyse, et peuvent être stockés. Ces antisérums peuvent être sous forme cristalline ou sous la forme de pulvérisation déposée sur les parois intérieures des cavités 22 ou sous forme liquide.

La fig. 3 illustre une autre forme de réalisation des supports d'analyse réalisés comme précédemment au moyen d'une feuille thermoplastique 30, dans laquelle sont ménagées des cavités 32 destinées à contenir les antisérums servant de réactifs, et des cavités allongées 33 servant de récipient d'analyse. Ce dispositif diffère de celui de la fig. 2 par l'absence des cavités 21 contenant les échantillons de sang, et par le fait que seule la rangée de cavités 32 est operculée par un film étanche 34. Dans cet exemple, les échantillons de sang sont amenés par des moyens quelconques, notamment par des aiguilles reliées à des pompes décrites plus en détail par la suite.

La fig. 4 représente une variante de réalisation du support d'analyse des fig. 2 et 3. Ce support comporte une feuille 40 en un matériau thermoplastique inerte au sang et aux réactifs, dans laquelle sont ménagées une cavité 41 sous la forme d'une goulotte allongée transversalement par rapport à la direction longitudinale de la feuille 40 et destinée à contenir le sang du patient, et une rangée de cavités 42 contenant les antisérums utilisés pour les réactions d'agglutination, et une série de cavités 43 en forme de canaux parallèles sert de cavités d'analyse dans lesquelles on ménage, comme précédemment, les échantillons de sang et les réactifs appropriés. Un film 44 recouvre de façon étanche la plaque 40 et les cavités 41, 42 et 43.

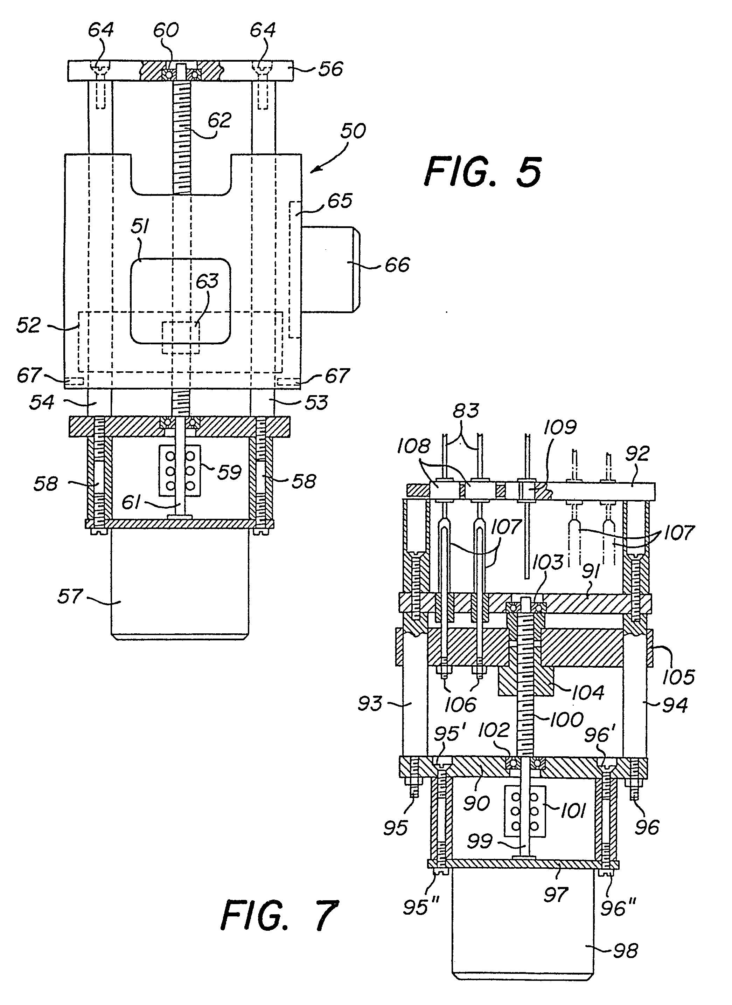

Ces supports d'analyse sont disposés à l'intérieur de l'appareil, sur une plaque dite plaque porte-support d'analyse, décrite plus en détail en référence à la fig. 5. La plaque porte-support d'analyse 50 est constituée de préférence par une plaque métallique, destinée à recevoir les supports d'analyse illustrés en référence aux fig. 2 à 4, et dans laquelle est ménagée une ouverture centrale 51 qui permet l'analyse par transparence des mélanges échantillons de sang et réactifs des supports d'analyse placés sur cette plaque. La plaque 50 est de préférence fixée sur une console 52 coulissant sur deux tiges 53 et 54 servant de rails de guidage, montées horizontalement entre deux plaques-supports 55 et 56. La plaque 55 sert par ailleurs de support à un moteur d'entraînement 57 monté sur la plaque 55 par l'intermédiaire de vis 58. Deux paliers à billes 59 et 60, représentés schématiquement, soutiennent l'axe moteur 61 prolongé par une vis sans fin 62, engagée à travers un bloc fileté 63 solidaire de la console 52. Deux vis 64 assurent la liaison entre la plaque de montage 56 et les tiges 53 et 54. Le moteur d'entraînement 57 engendre une rotation de la vis sans fin 62, ce qui assure le déplacement de la console 52, et par suite de la plaque porte-support d'analyse 50, pour amener successivement les supports d'analyse dans les différentes positions de traitement, c'est-à-dire successivement sous les aiguilles et sous le dispositif d'analyse colorimétrique. Une came rotative 65, couplée à un moteur électrique 66, peut être montée sur l'appareil solidaire de la console 52, pour assurer un mouvement de balancement autour d'un axe horizontal, ce mouvement ayant pour but de favoriser le mélange de sang et de réactifs et de faciliter le processus d'agglutination.

La plaque porte-support d'analyse 50 est de préférence recouverte par un couvercle (non représenté), articulé sur cette plaque par l'intermédiaire de goupilles logées dans les dégagements 67. Ce couvercle est prévu pour être rabattu sur la plaque 50, lorsque le support d'analyse est en place, et assure le blocage en position de ce support.

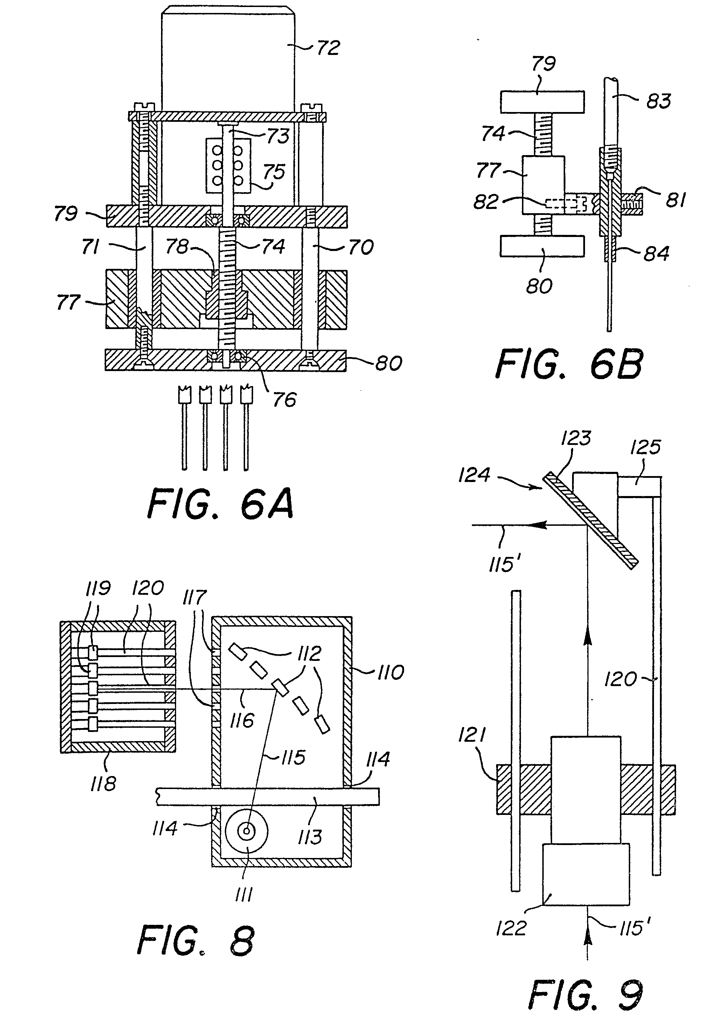

Les fig. 6A et 6B représentent respectivement deux vues en élévation de face et de profil du bloc portant les aiguilles de distribution pour prélever les échantillons de sang ou les réactifs, et les déposer dans les cavités d'analyse. Ce bloc se compose essentiellement d'un support formé de deux colonnes 70 et 71 portant un moteur d'entraînement 72 dont l'arbre de sortie 73 est couplé à une

vis sans fin 74, soutenue par deux paliers à billes 75 et 76. Un support 77 est monté sur un bloc fileté 78, de telle manière qu'il puisse coulisser verticalement le long des colonnes 70 et 71, lorsque le moteur d'entraînement 72 fait tourner la vis sans fin 74 dans un sens ou dans l'autre. Le support 77 peut être déplacé entre les deux plaques 79 et 80 entre lesquelles sont montées les colonnes 70 et 71. Il porte une pièce 81, fixée par exemple par des vis 82 et constituant le support des aiguilles 84. Ces aiguilles sont reliées chacune par l'intermédiaire d'un conduit souple 83 à l'une des pompes de prélèvement du bloc de pompes décrit plus en détail ci-dessous.

La fig. 7 illustre plus en détail le bloc de pompes, relié par l'intermédiaire de tubes souples aux aiguilles 84 des fig. 6A et 6B. Le bloc de pompes comporte à nouveau un support comprenant essentiellement les plates-formes horizontales 90, 91 et 92 et deux colonnes verticales 93 et 94. Sur la plate-forme 90 sont adaptées deux colonnes 95 et 96 au moyen de vis 95' et 95'' et 96' et 96''. Une plaque-support 97 permet le montage d'un moteur pas-à-pas 98 sur les colonnes 95 et 96. L'arbre de sortie 99 du moteur 98 est connecté à une vis sans fin 100, ces éléments étant supportés par les paliers à roulements à billes 101, 102 et 103.

Sur la vis sans fin 100 est monté un bloc fileté 104 solidaire d'un support 105 coulissant sur les colonnes 93 et 94. Ce support 105 permet le montage des pistons 106, des pompes 107 constituées par des tubes cylindriques montés entre les plates-formes 91 et 92, et à l'intérieur desquelles peuvent se déplacer les pistons correspondants 106. Des vannes électromagnétiques à trois voies 108 sont fixées sur la plate-forme 92, et ont une sortie supérieure connectée aux tubulures 83 flexibles qui sont reliées aux aiguilles 84 correspondantes (voir fig. 6B). Le dispositif comporte de préférence quatre pompes 107 et quatre vannes électromagnétiques 108 correspondant respectivement aux quatre aiguilles 84 décrites précédemment. Une vanne à bascule centrale 109 permet de prélever le liquide de dilution constitué de façon connue en soi par de l'eau déminéralisée mélangée à un agent détergent, ce liquide de dilution étant par la suite refoulé dans les canaux pour assurer le nettoyage du circuit.

La fig. 8 est une vue en élévation du dispositif colorimétrique permettant d'analyser les échantillons de sang traités au moyen des réactifs d'agglutination.

En référence à la fig. 8, le dispositif d'analyse colorimétrique se compose essentiellement d'un boîtier 110, à l'intérieur duquel est montée une source de lumière, par exemple une lampe à filament 111, et une série de miroirs plans 112 dont le nombre correspond au nombre de canaux du support d'analyse contenant la matière à analyser. Le support porte-plaques 113 traverse le boîtier 110 par deux fentes 114, de manière à couper le trajet du faisceau lumineux d'analyse 115. Ce faisceau est réfléchi sur l'un des miroirs 112, sous la forme d'un faisceau 116 qui traverse l'un des orifices 117 ménagés sur une face latérale du boîtier, pour pénétrer dans un boîtier 118 dans lequel sont logés des détecteurs au silicium 119. Le nombre de ces cellules est égal au nombre d'orifices 117 et au nombre de miroirs 112. En face de chaque orifice 117 est monté un élément tubulaire 120 logé dans le boîtier 118, pour guider l'un des faisceaux réfléchis 116. La mesure colorimétrique s'effectue par transparence. En l'absence d'agglutination du sang par l'effet d'un réactif, aucun signal n'est détecté sur les cellules au silicium. Par contre, lorsqu'il y a agglutination, le faisceau traverse le mélange contenu dans le canal correspondant du support d'analyse, et engendre un signal de la cellule au silicium.

Pour affiner la détection, on dispose de préférence une plaque transparente portant une trame perpendiculaire à la direction longitudinale des canaux du support d'analyse, cette trame étant constituée par une série de lignes parallèles très fines portées par l'une des surfaces de la plaque transparente. Cette plaque est disposée de préférence en dessous du support d'analyse. En l'absence d'agglutination, le dispositif d'analyse colorimétrique ne détecte pas la trame. Par contre, celle-ci est détectée par les cellules au silicium et engendre des pics sur le graphique lors du balayage du support d'analyse par le faisceau lumineux 115.

Un épiscope, décrit en référence à la fig. 9, permet de réfléchir l'image du support d'analyse, et notamment de la plage correspondant aux cavités d'analyse sous forme de canaux de ces supports. L'épiscope de la fig. 9 se compose essentiellement d'un support 120, comportant au moins deux colonnes verticales sur lesquelles peut coulisser un élément 121 portant un objectif 122. Cet objectif est disposé sur le trajet d'un faisceau lumineux 115' et focalise ce faisceau sur la surface réfléchissante 123 d'un miroir plan 124 relié par un organe de liaison 125 au support 120. Le faisceau 115', réfléchi par la surface 123, se projette sur un écran, par exemple un verre dépoli, monté à l'intérieur du boîtier 11 (voir fig. 1A) du dispositif photographique. De cette manière, on peut visualiser directement le support d'analyse, et notamment la plage correspondant aux cavités d'analyse de ces supports.

L'épiscope est disposé derrière le dispositif colorimétrique. Le support d'analyse est tout d'abord amené au-dessus du dispositif d'analyse colorimétrique, puis au-dessus de l'épiscope qui renvoie l'image de ce support sur l'écran d'affichage du dispositif photographique.

L'appareil décrit opère de la façon suivante: après mise en place des supports d'analyse, l'échantillon de sang est déposé manuellement dans la cavité correspondante. Les pompes servent à prélever des échantillons de sang et les antisérums correspondants servant de réactifs d'agglutination, et à refouler ces mélanges vers les aiguilles correspondantes pour les déposer dans les cavités d'analyse. Par la suite, un liquide de dilution, composé de préférence d'eau déminéralisée et d'un agent détergent, assure le rinçage du dispositif après chaque cycle d'utilisation. Les commandes d'ouverture et de fermeture des différentes voies sont assurées par les vannes commandées à trois voies.

Pour faciliter les réactions d'agglutination et notamment la réaction qui permet la détermination du facteur rhésus, les aiguilles sont partiellement logées dans des manchons chauffants destinés à maintenir les mélanges de sang et de réactifs à une température adéquate voisine de 37°C. Dans ce but, les manchons sont équipés de petites résistances électriques, connectées à un dispositif thermostatique.

## Revendications

1. Appareil pour déterminer le groupe sanguin d'un individu, comportant un support d'analyse individuel transparent pourvu d'une rangée transversale de cavités scellées renfermant des réactifs liquides d'agglutination et monté sur une plaque porte-support, un dispositif équipé de pompes et d'un groupe mobile d'aiguilles de distribution pour prélever des échantillons de sang et les mélanger en quantités déterminées avec lesdits réactifs, des organes mécaniques pour assurer le déplacement des aiguilles de distribution, des organes d'analyse photométrique pour détecter la présence ou l'absence d'une réaction d'agglutination entre les échantillons sanguins et les réactifs, et un dispositif pour afficher les résultats de cette analyse, caractérisé en ce que le support d'analyse individuel comporte une rangée transversale de cavités d'analyse (23, 43), dont chacune est alignée longitudinalement avec l'une desdites cavités pour réactifs (22, 42), et au moins une cavité pour le sang (21, 41), en ce que la plaque porte-support (50) est mobile horizontalement dans la direction longitudinale du support d'analyse et le groupe d'aiguilles de distribution (84) est mobile uniquement verticalement pour prélever le sang et les réactifs dans les cavités respectives et les déposer dans les cavités d'analyse, et en ce que les organes d'analyse photométrique comprennent un dispositif d'analyse colorimétrique comportant une série de détecteurs (119) associés à des faisceaux lumineux respectifs qui traversent lesdites cavités d'analyse pendant que le support d'analyse est en déplacement.

2. Appareil selon la revendication 1, caractérisé en ce que les cavités d'analyse (23, 43) ont la forme de canaux allongés, dont l'axe longitudinal passe sensiblement par le centre des cavités pour réactifs (22, 42).

3. Appareil selon la revendication 2, caractérisé en ce que la cavité pour le sang (41) présente une forme allongée s'étendant sensiblement sur toute la longueur de la rangée de cavités pour réactifs.

4. Appareil selon l'une quelconque des revendications 1 à 3, caractérisé en ce que le support d'analyse comporte une feuille de matière thermoplastique (20, 40) pourvue desdites cavités et une pellicule d'occlusion (24, 44) obturant hermétiquement au moins les cavités renfermant les réactifs.

5. Appareil selon la revendication 1, caractérisé en ce que la plaque porte-support (50) est connectée à une came (65) entraînée par un moteur (66), de telle manière qu'elle puisse effectuer un mouvement de balancement autour d'un axe horizontal.

6. Appareil selon la revendication 2, caractérisé en ce que le dispositif d'analyse colorimétrique comporte une plaque transparente portant une trame de lignes sombres parallèles, disposées perpendiculairement par rapport à la direction longitudinale des canaux qui constituent les cavités d'analyse (23, 43) du support d'analyse.

7. Appareil selon la revendication 1, caractérisé en ce que les aiguilles de distribution (84) comportent un manchon chauffant qui entoure au moins partiellement les pointes des aiguilles, ce manchon étant adapté pour maintenir les échantillons de sang à une température voisine de 37°C.

8. Appareil selon la revendication 1, caractérisé en ce qu'il comporte un épiscope pour renvoyer l'image des supports d'analyse sur un écran d'affichage.

9. Appareil selon la revendication 1, caractérisé en ce qu'il comporte un enregistreur graphique pour enregistrer, sous la forme de graphiques, les résultats de l'analyse colorimétrique.

## Patentansprüche

1. Gerät zur Bestimmung der Blutgruppe einer Person, das einen individuellen, transparenten Analyseträger umfasst, der mit einer transversalen Reihe von verschlossenen, Agglutinations-Reagenzstoffe enthaltenden Vertiefungen versehen und auf einer Trägerauflageplatte gelagert ist, sowie eine mit Pumpen und einer beweglichen Gruppe von Verteilnadeln ausgestattete Vorrichtung um Blutmuster zu entnehmen und sie mit bestimmten Mengen der genannten Reagenzstoffe zu vermischen, mechanische Mittel um die Bewegung der Verteilnadeln zu gewährleisten, photometrische Analysemittel zur Anzeige der Anwesenheit oder Abwesenheit einer Agglutinationsreaktion zwischen den Blutmustern und den Reagenzstoffen, und eine Vorrichtung zur Anzeige der Ergebnisse dieser Analyse, dadurch gekennzeichnet, dass der individuelle Analyseträger eine transversale Reihe von Analysevertiefungen (23, 43) aufweist, die jeweils in Längsrichtung ausgerichtet ist mit einer der genannten Vertiefungen für die Reagenzstoffe, und mindestens einer Vertiefung für das Blut (21, 41), dass die Trägerauflageplatte (50) in der Längsrichtung des Analyseträgers horizontal beweglich ist und die Verteilnadelgruppe (84) ausschliesslich vertikal beweglich ist um das Blut und die Reagenzstoff den entsprechenden Vertiefungen zu entnehmen und sie in den Analysevertiefungen abzulegen, und dass die photometrischen Analysemittel eine Vorrichtung zur kolorimetrischen Analyse umfassen, die eine Reihe von Detektoren aufweist, die entsprechenden Lichtbündeln zugeordnet sind, wobei diese die genannten Analysevertiefungen während der Bewegung des Analyseträgers durchsetzen.

2. Gerät nach Anspruch 1, dadurch gekennzeichnet, dass die Analysevertiefungen (23, 43) die Form von langestreckten Rinnen haben, deren Längsachse im wesentlichen durch die Mitte der Vertiefungen für die Reagenzstoffe (22, 42) hindurchgeht.

3. Gerät nach Anspruch 2, dadurch gekennzeichnet, dass die Vertiefung für das Blut (41) eine langgestreckte Form aufweist, die sich im wesentlichen über die gesamte Länge der Vertiefungen für die Reagenzstoffe erstreckt.

4. Gerät nach irgend einem der Ansprüche 1 bis 3, dadurch gekennzeichnet, dass der Analyseträger ein Blatt aus thermoplastischem Material (20, 40) umfasst, das mit den genannten Vertiefungen und einem Verschlussüberzug (24, 44) versehen ist, der mindestens die die Reagenzstoffe enthaltenden Vertiefungen verschliesst.

5. Gerät nach Anspruch 1, dadurch gekennzeichnet, dass die Trägerauflageplatte (50) mit einer Nocke (65) verbunden ist, die durch einen Motor (66) angetrieben ist, sodass sie eine Schaukelbewegung um eine horizontale Achse ausführen kann.

6. Gerät nach Anspruch 2, dadurch gekennzeichnet, dass die Vorrichtung zur kolorimetrischen Analyse eine durchsichtige Platte aufweist, die ein Raster von dunklen, parallelen Linien trägt, die senkrecht inbezug auf die Längsrichtung der Kanäle angeordnet sind, die die Analyseveriefungen (23, 43) des Analyseträgers bilden.

7. Gerät nach Anspruch 1, dadurch gekennzeichnet, dass die Verteilnadeln einen Heizmantel umfassen, der mindestens teilweise die Spitzen der Nadeln umgibt, wobei dieser Mantel vorgesehen ist um die Blutmuster bei einer Temperatur in der Nähe von 37°C zu erhalten.

8. Gerät nach Anspruch 1, dadurch gekennzeichnet, dass es episkopische Mittel zur Rückleitung des Bildes des Analyseträgers zu einem Anzeigeschirm aufweist.

9. Gerät nach Anspruch 1, dadurch gekennzeichnet, dass es ein graphisches Registrierinstrument umfasst, das zum reigstrieren der Ergebnisse der kolorimetrischen Analyse in Form einer graphischen Darstellung vorgesehen ist.

## Claims

1. Apparatus for determining the blood group of an individual, comprising a transparent analysis holder provided with a cross row of sealed cavities adapted to receive liquid agglutination reactants, and mounted on a carrier plate, pumping means associated with a block of distribution needles, for displacing blood samples and reactants to form analyzable mixture, mechanical means for displacing the distribution needles, photometrical analysis means for detecting the presence or absence of an agglutination reaction between blood samples and reactants and means for displaying the results of the analysis, characterized in that the analysis cavities (23, 43) are situated in a row accross the analysis holder, each analysis cavity being on a center line with a corresponding reactant cavity (22, 42) and at least one blood sample cavity (21, 41), in that the carrier plate (50) is slidably mounted for horizontal motion in the longitudinal direction of the holder analysis, and the block of distribution needles (84) is slidably mounted for only vertical movement so as to cause blood samples and reactants to be pumped from their corresponding cavities to analysis cavities, and in that the photometrical analysis means comprises colorimetrical analysis means provided with detector (119) corresponding respectively to the rays of light passing through said analysis cavities during the displacement of the analysis holder.

2. Apparatus according to claim 1, characterized in that the analysis cavities (23, 43) are in the form of elongated channels, each analysis cavity being on a center line with a corresponding reactant cavity (22, 42).

3. Apparatus according to claim 2, characterized in that the blood sample cavity (41) is in the form of an elongated channel which run longitudinally of the row of reactant cavities.

4. Apparatus according to any one of claims 1 to 3, characterized in that the analysis holder comprises a sheet (20) of thermoplastic material provided with said cavities and a covering sheet (24, 44) sealing off at least cavities containing the reactants.

5. Apparatus according to claim 1, characterized in that a rotating cam (65) driven by a motor (66) is mounted to provide a rocking movement to carrier plate (5) about a horizontal axis.

6. Apparatus according to claim 2, characterized in that the colorimetrical analysis means are provided with a transparent plate carrying a grid of parallel dark lines arranged perpendicularly in relation to the longitudinal axes of analysis cavities (23, 43) of the analysis holder.

7. Apparatus according to claim 1, characterized in that the distribution needles (84) are at least partially secured in heater sleeves designed to keep the blood samples at a temperature approaching 37°C.

8. Apparatus according to claim 1, characterized in that it comprises an episcope allowing the image of the analysis holder to be reflected on a display screen.

9. Apparatus according to claim 1, characterized in that it comprises means provided for graphically representing the colorimetrical analysis results.

0 079 861

FIG. 1A

FIG. 1B

FIG. 2

FIG. 3

FIG. 4

7

FIG. 5

FIG. 7

0 079 861

FIG. 6A

FIG. 6B

FIG. 8

FIG. 9

11